# EUROPÄISCHE PATENTSCHRIFT

(11) **EP 3 180 052 B1**
(45) Veröffentlichungstag und Bekanntmachung des Hinweises auf die Patenterteilung: **08.05.2019**
(21) Anmeldenummer: 15747826.4
(22) Anmeldetag: 10.08.2015
(51) Int. Cl.: A61M 1/36, A61M 5/168, G08B 21/02

(54) **VERFAHREN ZUR ÜBERWACHUNG DER AUSRICHTUNG UND/ODER POSITIONIERUNG EINER BLUTSCHLAUCHLEITUNG EINES EXTRAKORPORALEN BLUTKREISLAUFS IN EINEM ZUGANGSBEREICH**
METHOD FOR MONITORING THE ALIGNMENT AND/OR POSITIONING OF A BLOOD TUBE LINE OF AN EXTRACORPOREAL BLOOD CIRCUIT IN AN ACCESS REGION
PROCÉDÉ DE SURVEILLANCE DE L'ALIGNEMENT ET/OU DU POSITIONNEMENT D'UNE TUBULURE FLEXIBLE SANGUINE D'UNE CIRCULATION SANGUINE EXTRACORPORELLE DANS UNE ZONE D'ACCÈS

(30) Priorität: 11.08.2014 DE 102014111403
(43) Veröffentlichungstag der Anmeldung: 21.06.2017
(73) Patentinhaber: Fresenius Medical Care Deutschland GmbH, 61352 Bad Homburg (DE)
(72) Erfinder: SPICKERMANN, Reiner, 97535 Wasserlosen-Burghausen (DE)
(74) Vertreter: Bobbert & Partner Patentanwälte PartmbB
(86) Internationale Anmeldenummer: PCT/EP2015/068389
(87) Internationale Veröffentlichungsnummer: WO 2016/023868

(56) Entgegenhaltungen:
- EP-A1- 1 892 001
- EP-A2- 1 574 178
- EP-A2- 2 446 909
- WO-A1-2011/123624
- US-A1- 2003 128 125
- US-A1- 2009 080 757

## Beschreibung

Die vorliegende Erfindung betrifft ein Verfahren zur Überwachung der Ausrichtung und/oder Positionierung wenigstens einer Blutschlauchleitung eines extrakorporalen Blutkreislaufs in einem Zugangsbereich gemäß Anspruch 1. Die vorliegende Erfindung betrifft ferner ein System nach Anspruch 12 und eine Blutbehandlungsvorrichtung nach Anspruch 13.

In der medizinischen Praxis, insbesondere im Bereich der extrakorporalen Blutbehandlung, gilt es ungewollte Nadeldiskonnektionen während einer Behandlung, welche schwere Folgen für den Patienten haben können, unbedingt zu vermeiden. In diesem Zusammenhang sind Vorrichtungen und Systeme bekannt, welche die Erkennung einer Blutung während einer solchen Blutbehandlung ermöglichen.

Die US 2009/0080757 A1 offenbart ein Verfahren zum Erkennen bzw. Überwachen eines Blutaustritts eines Patientenzugangsbereiches. Das Verfahren verwendet eine Wärmebild- oder Infrarotkamera, wobei wenigstens zwei Aufnahmen des Zugangsbereichs verarbeitet und verglichen werden, um eine Entstehung bzw. Veränderung eines Blutflecks nachzuweisen und ein entsprechendes Überwachungssignal zu erzeugen.

Die EP 1 892 001 A1 offenbart ein medizinisches Gerät zur extrakorporalen Blutbehandlung mit einer Farbbildkamera zur Erkennung der Farbe von Blut.

Die US 2003/0128125 A1 offenbart ein Verfahren zum Erkennen eines Verlusts der Integrität eines extrakorporalen Blutkreislaufs.

Die EP 2 446 909 A2 offenbart eine Sicherungseinrichtung zur Kontrolle eines Gefäßzugangs eines Lebewesens.

Die EP 1 575 178 A2 offenbart ein medizinisches Behandlungssystem mit mindestens einem Behandlungsplatz.

Die WO 2011/123624 A1 offenbart ein Verfahren zum Bestimmen einer Position einer Flüssigkeitsleckage.

Eine Aufgabe der vorliegenden Erfindung ist es, insbesondere ein Verfahren und Vorrichtungen anzugeben, welche das mit einer Nadeldiskonnektion verbundene Risiko einer Blutung während einer medizinischen Blutbehandlung verringern bzw. minimieren.

Die erfindungsgemäße Aufgabe wird durch ein Verfahren mit den Merkmalen des Anspruchs 1 gelöst. Die erfindungsgemäße Aufgabe wird ferner durch ein System nach Anspruch 12 und eine Blutbehandlungsvorrichtung nach Anspruch 13 gelöst.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenwerte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst, wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wann immer hierin der Ausdruck "konfiguriert", "programmiert", "ausgebildet" oder "ausgestaltet" verwendet wird, so versteht der Fachmann diesen als "ausgebildet, ausgestaltet, programmiert und/oder konfiguriert", wo immer dies für den Fachmann erkennbar technisch möglich und/oder sinnvoll ist.

Der Erfindung liegt die Erkenntnis zugrunde, dass einer Nadeldiskonnektion eine, gegebenenfalls auffällige, Änderung der Ausrichtung und/oder Positionierung zumindest eines Teils oder Abschnitts wenigstens einer Blutschlauchleitung im Zugangsbereich vorausgeht.

Erfindungsgemäß wird somit ein Verfahren vorgeschlagen, mit welchem die Ausrichtung, und/oder die Positionierung, wenigstens einer Blutschlauchleitung oder wenigstens eines Teiles oder Abschnitts einer Blutschlauchleitung in einem Zugangsbereich überwacht wird. Dabei ist die wenigstens eine Blutschlauchleitung eine Blutschlauchleitung eines extrakorporalen Blutkreislaufs und mit einer arteriellen und/oder einer venösen Patientennadel verbunden. Weiterhin umfasst das Verfahren wenigstens das Bereitstellen wenigstens einer Wärmebild- oder Infrarotkamera zur Beobachtung der Temperaturverteilung der Oberfläche des Zugangsbereichs, das Aufnehmen wenigstens eines ersten Bildes des Zugangsbereichs als Referenzbild und das Aufnehmen wenigstens eines zusätzlichen Bildes des Zugangsbereichs. Weiterhin umfasst das Verfahren das Verarbeiten des wenigstens einen Referenzbildes und des wenigstens einen zusätzlichen Bildes mittels einer Recheneinheit, welche wenigstens konfiguriert ist, um erste Daten aus dem Referenzbild als Referenzdaten und zusätzliche Daten aus dem zusätzlichen Bild zu erzeugen. Dabei werden, insbesondere optional, zumindest die Referenzdaten und/oder das Referenzbild gespeichert. Zusätzlich ist die Recheneinheit konfiguriert, um aus den Referenzdaten und den zusätzlichen Daten eine Veränderung der Ausrichtung und/oder der Positionierung wenigstens eines Teils oder Abschnitts der Blutschlauchleitung im Zugangsbereich nachzuweisen und ein entsprechendes Überwachungssignal zu erzeugen oder zu verändern.

Weiterhin wird ein erfindungsgemäßes System mit wenigstens einer Wärmebild- oder Infrarotkamera vorgeschlagen, welches wenigstens eine zur Ausführung eines erfindungsgemäßen Verfahrens konfigurierte Recheneinheit aufweist.

Zudem wird eine erfindungsgemäße Blutbehandlungsvorrichtung vorgeschlagen, welche wenigstens ein erfindungsgemäßes System aufweist.

Erfindungsgemäße Ausführungsformen der vorliegenden Erfindung können ein oder mehrere der im Folgenden genannten Merkmale in beliebiger Kombination aufweisen, sofern die konkrete Kombination nicht für den Fachmann erkennbar technisch unmöglich ist.

Vorteilhafte Weiterentwicklungen der vorliegenden Erfindung sind jeweils Gegenstand von Unteransprüchen und Ausführungsformen.

Unter "Blutschlauchleitung" kann im Folgenden wenigstens ein Teil oder Abschnitt einer Schlauchleitung, insbesondere eines extrakorporalen Blutkreislaufs, verstanden werden, welches vorgesehen und/oder ausgestaltet ist, um mit einem Blutgefäßsystem, beispielsweise mittels einer Patientennadel, verbunden und/oder verbindbar zu sein.

Unter "Zugangsstelle" kann die Stelle eines Zugangs in einem Zugangsbereich zu einem Blutgefäßsystem eines Lebewesens, insbesondere eines Menschen, beispielsweise eines arteriellen und/oder venösen Zugangs, verstanden werden.

Unter "Zugangsbereich" kann ein Bereich um eine Zugangsstelle oder ein Bereich, der wenigstens eine Zugangsstelle umfasst, beispielsweise einen Überwachungsbereich der Zugangsstelle, verstanden werden.

Unter "Nachweisen einer Veränderung" kann die Feststellung und/oder eine Quantifizierung einer Veränderung verstanden werden. Beispielsweise kann der Nachweis einer Veränderung voraussetzen, dass die Veränderung zum einen festgestellt wurde und/oder zum anderen einen vorbestimmten oder vorbestimmbaren Grenzwert überschreitet.

"Wärmebildkamera oder Infrarotkamera", auch bekannt als Thermographie- oder Thermalkamera oder als Wärmebildgerät, bezeichnet hierhin eine bildgebende Vorrichtung, welche zur Messung der Intensitätsverteilung infraroter Strahlung, Wärmestrahlung oder der Temperaturverteilung der Oberfläche eines Körpers oder Objekts und zur Erzeugung entsprechender, insbesondere darstellbarer, Bilddaten dient.

Unter "Ausrichtung" kann insbesondere eine räumliche Ausrichtung verstanden werden. Unter "Positionierung" kann insbesondere eine räumliche Positionierung verstanden werden.

In manchen erfindungsgemäßen Ausführungsformen kann die Patientennadel eine Einnadel (engl.: "single needle") oder eine Dualnadel (engl.: "double needle") sein.

In einigen erfindungsgemäßen Ausführungsformen des Verfahrens ist der extrakorporale Blutkreislauf ein Blutkreislauf einer Blutbehandlungsvorrichtung oder ist mit einer solchen verbunden und/oder verbindbar.

In manchen erfindungsgemäßen Ausführungsformen umfasst der Zugangsbereich zumindest eine Zugangsstelle des extrakorporalen Blutkreislaufs zum Gefäßsystem eines Patienten.

In bestimmten erfindungsgemäßen Ausführungsformen ist der wenigstens eine Teil oder Abschnitt der Blutschlauchleitung ein Abschnitt, insbesondere von vorgegebener und/oder vorgebbarer Länge, welcher sich in unmittelbarer Nähe der Zugangsstelle befindet und welcher beispielsweise einseitig mit einer Patientennadel verbunden ist.

In einigen erfindungsgemäßen Ausführungsformen des Verfahrens wird wenigstens eine Wärmebild- oder Infrarotkamera zur Beobachtung und/oder Überwachung des Zugangsbereichs angebracht und/oder ausgerichtet.

In bestimmten erfindungsgemäßen Ausführungsformen des Verfahrens ist die Temperaturverteilung durch die Verteilung der Wärmestrahlungsintensität der Oberfläche des Zugangsbereichs ermittelbar.

In manchen erfindungsgemäßen Ausführungsformen umfasst das Verfahren das Aufnehmen wenigstens eines ersten Bildes des Zugangsbereichs als Referenzbild. Dies geschieht beispielsweise am Anfang einer Blutbehandlung und/oder wenigstens zu einem Zeitpunkt der Behandlung, bei welchem die Blutschlauchleitung oder Blutschlauchleitungen in einem korrekten, insbesondere funktionsfähigen Zugangszustand im Zugangsbereich derart angeordnet und/oder angebracht sind, dass der extrakorporale Blutkreislauf und ein Blutgefäßsystem verbunden sind.

In einigen erfindungsgemäßen Ausführungsformen des Verfahrens erfolgt das Aufnehmen des wenigstens einen zusätzlichen Bildes des Zugangsbereichs, insbesondere nach der Aufnahme des Referenzbildes.

Unter "Bild" oder "Referenzbild" können im Folgenden Bilddaten, beispielsweise Grauwert- und/oder binäre Bilddaten, verstanden werden, welche der Intensitätsverteilung infraroter Strahlung und/oder der Temperaturverteilung auf der Oberfläche des beobachteten und/oder überwachten Zugangsbereichs entsprechen, diese repräsentieren und/oder beinhalten.

In manchen erfindungsgemäßen Ausführungsformen umfasst das Verfahren optional wenigstens die Speicherung der Referenzdaten, des Referenzbildes, der zusätzlichen Daten und/oder des wenigstens einen zusätzlichen Bildes.

In einigen erfindungsgemäßen Ausführungsformen enthält das Überwachungssignal wenigstens die, beispielsweise binäre, Information, ob eine Änderung der Ausrichtung und/oder der Positionierung wenigstens einer Blutschlauchleitung, oder eines Teiles oder Abschnitts hiervon, im Zugangsbereich nachgewiesen oder ausgeschlossen wurde.

In bestimmten erfindungsgemäßen Ausführungsformen erfolgt die Überwachung und/oder die Aufnahme des Referenzbildes und/oder der zusätzlichen Bilder während einer Blutbehandlung, insbesondere während einer extrakorporalen Blutbehandlung.

In manchen erfindungsgemäßen Ausführungsformen umfasst die Recheneinheit wenigstens eine Bildverarbeitungssoftware, insbesondere zur Verarbeitung des Referenzbildes und/oder der zusätzlichen Bilder und/oder zur Erzeugung von Referenzdaten aus dem wenigstens einen Referenzbild und/oder von zusätzlichen Daten aus dem wenigstens einen zusätzlichen Bild.

In einigen erfindungsgemäßen Ausführungsformen ist die Bildverarbeitungssoftware zumindest zur oder ausschließlich zur Verarbeitung von Grauwert-Bilddaten konfiguriert.

In manchen erfindungsgemäßen Ausführungsformen kann wenigstens eine zusätzliche Bildaufnahmevorrichtung, beispielsweise eine digitale Kamera, mit der Wärmebild- und/oder Infrarotkamera synchronisiert, auf diese abgestimmt oder abstimmbar sein. Dies kann beispielsweise in der Art geschehen, dass stets der gleiche Zugangsbereich von beiden Kameras, oder zumindest ein bestimmter Abschnitt des von der Wärmebild- oder Infrarotkamera beobachteten oder überwachten Zugangsbereichs, von der zusätzlichen Bildaufnahmevorrichtung beobachtet und/oder überwacht wird.

In bestimmten erfindungsgemäßen Ausführungsformen kann die Bildverarbeitungssoftware zusätzlich zur Verarbeitung anderer Bilddatenformate, beispielsweise Farbbildformate konfiguriert sein. Hierzu zählen insbesondere solche, die mit einer zusätzlichen Bildaufnahmevorrichtung, beispielsweise einer digitalen Kamera, aufgenommen worden sind oder werden.

In einigen erfindungsgemäßen Ausführungsformen umfasst die Verarbeitung des Referenzbildes und/oder der zusätzlichen Bilder, Muster- und/oder Objekterkennung, Formanalyse, Formextraktion, Regionenerkennung und/oder Kantenerkennung und/oder die Bildverarbeitungssoftware ist hierzu konfiguriert.

In bestimmten erfindungsgemäßen Ausführungsformen beinhaltet die Muster-, Objekt-, Regionen- und/oder Kantenerkennung keine Erkennung von Blutmuster(n) und/oder die Bildverarbeitungssoftware und/oder die Recheneinheit ist nicht dazu ausgebildet, konfiguriert und/oder programmiert.

In manchen erfindungsgemäßen Ausführungsformen ist die Recheneinheit wenigstens zur Erkennung eines in einem Bild enthaltenen Musters, insbesondere eines Temperaturmusters, ausgebildet, konfiguriert und/oder programmiert.

In einigen erfindungsgemäßen Ausführungsformen kann ein Muster beispielsweise das Referenzbild, ein Abschnitt oder eine vorbestimmte oder vorbestimmbare Region hiervon sein.

In bestimmten erfindungsgemäßen Ausführungsformen ist die Recheneinheit wenigstens zur Erkennung eines im Bild enthaltenen Objektes, insbesondere eines vorgegebenen, vorgebbaren und/oder eines in einem Referenzbild zuvor erkannten Objektes, beispielsweise eines Referenzobjektes oder Referenzelementes, ausgebildet, konfiguriert und/oder programmiert.

In manchen erfindungsgemäßen Ausführungsformen ist ein Objekt insbesondere eine, beispielsweise zusammenhängende, Fläche oder ein Ensemble von Bildpunkten, welche beispielsweise Bildpunkte oder Pixel gleicher Temperatur oder die innerhalb eines vorbestimmten, vorbestimmbaren und/oder zu bestimmenden Temperaturbereichs fallen, umfasst.

In bestimmten erfindungsgemäßen Ausführungsformen kann ein Objekt insbesondere wenigstens eine Linie, eine Kante, und/oder eine Fläche, insbesondere bestimmter Form, und/oder eine Kombination hiervon sein. Beispielsweise können diese in einer bestimmten Ausrichtung und/oder in einem bestimmten Verhältnis, beispielsweise in einem bestimmten Flächen- und/oder Längenverhältnis, zueinander oder zu einem gemeinsamen Objekt, insbesondere zu einem Referenzelement, stehen.

In einigen erfindungsgemäßen Ausführungsformen ist eine Region eine vorgegebene oder vorgebbare Fläche, beispielsweise eine Fläche mit einem Objekt oder eine Fläche, um ein im Referenzbild erkanntes Objekt des Zugangsbereichs herum, welche insbesondere im Bezug auf ein Referenzelement und/oder einem bestimmten oder bestimmbaren Punkt oder Objekt des Bildes, insbesondere des Referenzbildes und des zusätzlichen Bildes, in einer bestimmten Ausrichtung und/oder Positionierung zu diesem steht.

In manchen erfindungsgemäßen Ausführungsformen kann eine Region, insbesondere ihre Fläche oder wenigstens eine ihrer Abmessungen, in einem bestimmten Längenverhältnis und/oder Flächenverhältnis zu einem Referenzelement stehen.

In bestimmten erfindungsgemäßen Ausführungsformen kann eine Region insbesondere ein Bereich des Zugangsbereichs sein, welcher ein bestimmtes Muster und/oder wenigstens ein bestimmtes Objekt, beispielsweise ein Referenzelement und/oder einen bestimmten Punkt einer Blutschlauchleitung wie eine Zugangsstelle, umfasst, umschließt und/oder enthält.

In einigen erfindungsgemäßen Ausführungsformen ist die Recheneinheit zur Ermittlung der Anzahl der Pixel oder Bildpunkte wenigstens einer vorgegebenen, vorgebbaren, zu erkennenden und/oder erkannten Region und/oder eines vorgegebenen, vorgebbaren, zu erkennenden und/oder erkannten Objekts, insbesondere eines Referenzbildes und/oder eines zusätzlichen Bildes, ausgebildet. Insbesondere kann die Recheneinheit zur Ermittlung der Anzahl der Bildpunkte oder Pixel gleicher oder identischer Temperaturen oder der Bildpunkte oder Pixel, die in einen vorbestimmten und/oder vorbestimmbaren Temperaturbereich fallen, konfiguriert sein.

In bestimmten erfindungsgemäßen Ausführungsformen kann eine Orientierungs- und/oder Skalierungsreferenz, beispielsweise ein Referenzelement, am oder im Zugangsbereich oder um den Zugangsbereich herum, beispielsweise in einem Bereich um die Zugangsstelle, angebracht werden oder zuvor angebracht worden sein, insbesondere vor der Überwachung und/oder vor einer Blutbehandlung.

In einigen erfindungsgemäßen Ausführungsformen ist das Referenzelement ein thermisches Element, insbesondere ein kühles, wärmeisolierendes, wärmeabweisendes oder ein warmes, wärmespeicherndes oder wärmerzeugendes Element, beispielsweise ein kühlbares oder erwärmbares Element, und/oder ein optisches Element, beispielsweise eine Markierung, das insbesondere im Zugangsbereich ausgebildet und/oder angebracht ist.

In manchen erfindungsgemäßen Ausführungsformen ist die Recheneinheit wenigstens zur Erkennung eines, insbesondere aufgebrachten, Referenzelements konfiguriert.

In einigen erfindungsgemäßen Ausführungsformen des Verfahrens ist die wenigstens eine Wärmebild- oder Infrarotkamera auf ein, insbesondere im Zugangsbereich angebrachtes, Referenzelement ausgerichtet und /oder auf das Referenzelement ausrichtbar angeordnet, beispielsweise mittels eines Antriebs und/oder einer Kipp- und/oder Schwenkvorrichtung.

In manchen erfindungsgemäßen Ausführungsformen erfolgt die Verarbeitung und/oder die Speicherung des Referenzbildes und/oder der zusätzlichen Bilder und/oder gegebenenfalls anderer mit zusätzlichen Bildaufnahmevorrichtungen aufgenommenen Bilder oder Bilddaten auf Basis von Grauwert-Daten. Insbesondere können Bilder und/oder Bilddaten beispielsweise als Grauwert-Daten gespeichert und/oder verarbeitet werden. Gegebenenfalls werden Bilder oder Bilddaten, die in einem anderen Format aufgenommen und/oder gespeichert wurden zuvor in Grauwert-Daten umgewandelt.

In bestimmten erfindungsgemäßen Ausführungsformen wird ein Überwachungssignal erst verändert und/oder erzeugt, wenn die Veränderung einen vorbestimmten oder vorbestimmbaren Grenzwert überschreitet.

In einigen erfindungsgemäßen Ausführungsformen des Verfahrens ist die Recheneinheit konfiguriert, um wenigstens ein entsprechendes Überwachungssignal zu erzeugen und/oder eine entsprechende Veränderung eines Überwachungssignals oder eines Zustands oder Inhalts eines Überwachungssignals zu bewirken, wenn ein Referenzelement nicht oder nur teilweise im Bild erkannt wird und/oder wenn nicht wenigstens ein zu erkennendes Objekt, oder wenigstens ein Teil oder Abschnitt hiervon, beispielsweise eine Blutschlauchleitung, im Zugangsbereich erkannt wird.

In bestimmten erfindungsgemäßen Ausführungsformen des Verfahrens kann wenigstens ein Überwachungssignal an wenigstens eine geeignete Einrichtung weitergeleitet oder übermittelt werden. Eine solche Einrichtung kann beispielsweise wenigstens zur Ausgabe eines wahrnehmbaren, beispielsweise hörbaren und/oder sichtbaren, Alarms und/oder zum Regeln und/oder Steuern, beispielsweise Anhalten oder Schließen, wenigstens einer Blutpumpe, wenigstens einer Substituatpumpe oder eines Substituatflusses und/oder einer Schlauchklemme ausgebildet und/oder konfiguriert sein.

In manchen erfindungsgemäßen Ausführungsformen des Verfahrens wird wenigstens ein Überwachungssignal an wenigstens eine Steuereinrichtung einer Blutbehandlungsvorrichtung, welche eine Blutpumpe aufweist oder betreibt, übermittelt. Die Steuereinrichtung kann beispielsweise zur Regelung und/oder Steuerung der Blutpumpe und/oder wenigstens eines Zustands einer arteriellen und/oder venösen Klemme der zugehörigen arteriellen und/oder venösen Blutschlauchleitung ausgebildet und/oder konfiguriert sein.

In einigen erfindungsgemäßen Ausführungsformen des Verfahrens kann wenigstens eine Steuereinrichtung vorzugsweise automatisch betrieben werden, insbesondere wenn ein entsprechendes Überwachungssignal vorliegt, ermittelt und/oder übermittelt wird und/oder wenn ein solches Überwachungssignal einen bestimmten, insbesondere im voraus definierbaren und/oder einstellbaren, Grenzwert über- oder unterschreitet.

In bestimmten erfindungsgemäßen Ausführungsformen des Verfahrens kann wenigstens ein von der Recheneinheit erzeugtes oder verändertes Überwachungssignal zusätzlich darstellbare Bilddaten des Bildes umfassen, bei welchem das Überwachungssignal erzeugt und/oder geändert wurde.

In einigen erfindungsgemäßen Ausführungsformen des Verfahrens kann das Überwachungssignal Bilddaten für die Darstellung des Referenzbildes und/oder wenigstens eines zusätzlichen, insbesondere aktuellen, Bildes umfassen. Beispielsweise können diese Bilddaten oder deren beliebige Überlagerungen und/oder Anordnungen auf wenigstens eine Bilddarstellungseinrichtung dargestellt werden.

In manchen erfindungsgemäßen Ausführungsformen des Verfahrens ist die Recheneinheit oder eine andere Vorrichtung zur Erzeugung von darstellbaren, farblichen Bilddaten konfiguriert.

In einigen erfindungsgemäßen Ausführungsformen weist eine Einrichtung, an welche das Überwachungssignal weitergeleitet oder übermittelt wird, insbesondere eine Alarmeinheit und/oder beispielsweise eine Bilddarstellungseinrichtung auf.

In bestimmten erfindungsgemäßen Ausführungsformen des Verfahrens kann die Feststellung oder der Nachweis einer Änderung der Ausrichtung und/oder Positionierung der Blutschlauchleitung, oder wenigstens eines Abschnitts hiervon, durch einen Vergleich der Referenzdaten mit den zusätzlichen Daten erfolgen.

In manchen erfindungsgemäßen Ausführungsformen des Verfahrens umfassen die Referenzdaten und/oder die zusätzlichen Daten wenigstens ein Muster, eine Anzahl von erkannten oder zu erkennenden Objekten, Abmessungen und/oder Flächen von erkannten und/oder zu erkennenden Objekten, und/oder eine relative Position und/oder Ausrichtung und/oder wenigstens einen Abstand zwischen wenigstens zwei erkannten oder zu erkennenden Objekten und/oder zwischen wenigstens einem Objekt und einem Referenzelement. Beispielsweise können die Referenzdaten und/oder die zusätzlichen Daten wenigstens die Ausrichtung und/oder Positionierung wenigstens einer Blutschlauchleitung, oder eines Teiles oder Abschnitts hiervon, bezüglich einer weiteren Blutschlauchleitung, oder eines Teiles oder Abschnitts hiervon, umfassen.

In manchen erfindungsgemäßen Ausführungsformen des Verfahrens kann in einem zusätzlichen Bild eine Veränderung der Ausrichtung und/oder Positionierung der Blutschlauchleitung durch die Veränderung der Position, der Ausrichtung, der Größe, der Form und/oder der Abmessung wenigstens eines im Referenzbild erkannten oder zu erkennenden Musters und/oder Objekts und/oder Region nachgewiesen werden.

In einigen erfindungsgemäßen Ausführungsformen des Verfahrens kann in einem zusätzlichen Bild eine Veränderung der Ausrichtung und/oder Positionierung der Blutschlauchleitung, oder wenigstens eines Abschnitts hiervon, durch eine Veränderung eines Abstands und/oder einer Ausrichtung zwischen zwei vorbestimmten, vorbestimmbaren und/oder zuvor erkannten Objekten nachgewiesen werden.

In manchen erfindungsgemäßen Ausführungsformen des Verfahrens kann in einem zusätzlichen Bild eine Veränderung der Ausrichtung und/oder Positionierung der Blutschlauchleitung, oder wenigstens eines Abschnitts hiervon, durch eine Veränderung der Ausrichtung und/oder Positionierung wenigstens einer vorbestimmten oder vorbestimmbaren Region und/oder Objekts im Bezug auf ein Referenzelement und/oder in Bezug auf ein erkanntes oder zu erkennendes Objekt nachgewiesen werden.

In bestimmten erfindungsgemäßen Ausführungsformen der vorliegenden Erfindung wird die Veränderung der Ausrichtung und/oder Positionierung wenigstens eines Abschnitts einer Blutschlauchleitung bezüglich eines Objektes, insbesondere eines Referenzobjektes oder Referenzelements, überwacht und/oder nachgewiesen.

In einigen erfindungsgemäßen Ausführungsformen des Verfahrens kann die Veränderung der Ausrichtung und/oder Positionierung der Blutschlauchleitung, oder wenigstens eines Abschnitts hiervon, durch eine Veränderung der Ausrichtung und/oder Positionierung wenigstens einer Blutschlauchleitung, oder eines Abschnitts hiervon, bezüglich einer weiteren Blutschlauchleitung, oder eines Abschnitts hiervon, nachgewiesen werden.

Die Erfindung betrifft weiterhin ein erfindungsgemäßes System mit wenigstens einer Wärmebild- oder Infrarotkamera und wenigstens einer Recheneinheit, welche zur Ausführung eines erfindungsgemäßen Verfahrens ausgebildet, programmiert und/oder konfiguriert ist.

Das erfindungsgemäße System weist insbesondere alle notwendigen Einrichtungen auf, welche zur Ausführung der jeweiligen erfindungsgemäßen Verfahrensschritte vorgesehen und/oder ausgebildet und/oder konfiguriert sind.

In einigen erfindungsgemäßen Ausführungsformen des Systems ist die Wärmebild- oder Infrarotkamera kabellos, beispielsweise per Funk, mit der Recheneinheit verbunden und/oder verbindbar.

In manchen erfindungsgemäßen Ausführungsformen ist das System als Zusatzsystem für wenigstens ein medizinisches Gerät und/oder für wenigstens einen Behandlungsraum ausgebildet.

In einigen erfindungsgemäßen Ausführungsformen umfasst das System weiterhin eine externe Einrichtung, welche insbesondere wenigstens zur Ausgabe eines wahrnehmbaren Alarms und/oder zur Bilddarstellung ausgebildet und/oder konfiguriert ist und/oder ist mit einer externen Einrichtung funktionell, insbesondere kabellos, beispielsweise per Funk, verbunden.

In manchen erfindungsgemäßen Ausführungsformen umfasst das System wenigstens einen Antrieb und/oder eine Kipp- und/oder Schwenkvorrichtung zur Ausrichtung, insbesondere Selbstausrichtung, der Wärmebild- oder Infrarotkamera und gegebenenfalls einer, beispielsweise damit synchronisierten zusätzlichen Bildaufnahmevorrichtung. Beispielsweise ist die Wärmebild- oder Infrarotkamera und/oder eine damit synchronisierte zusätzliche Bildaufnahmevorrichtung auf ein Referenzelement im Bereich der Zugangsstelle ausgerichtet und/oder ausrichtbar, insbesondere selbstausrichtend.

Schließlich betrifft die Erfindung eine Blutbehandlungsvorrichtung mit wenigstens einem erfindungsgemäßen System.

In manchen erfindungsgemäßen Ausführungsformen weist die Blutbehandlungsvorrichtung die zum Durchführen des erfindungsgemäßen Verfahrens erforderlichen und hierfür vorgesehenen und/oder konfigurierten Einrichtungen auf. Dies gilt insbesondere für die im Zusammenhang mit den hierin zum offenbarten Verfahren genannten Einrichtungen. Dabei kann die Blutbehandlungsvorrichtung - wie jede andere Vorrichtung gemäß der vorliegenden Erfindung - wenigstens eine oder mehrere geeignete Einrichtung(en) aufweisen, welche geeignet und/oder konfiguriert und/oder ausgestaltet ist oder sind, so dass einer, mehrere oder alle der hierin genannten Verfahrensschritte mittels der betreffenden Vorrichtung(en) durchgeführt werden können.

In manchen erfindungsgemäßen Ausführungsformen der Blutbehandlungsvorrichtung ist der extrakorporale Blutkreislauf mit einem Blutkreislauf eines Patienten mittels einer Einnadel oder einer Dualnadel verbindbar und/oder verbunden.

In einigen erfindungsgemäßen Ausführungsformen der Blutbehandlungsvorrichtung ist das System wenigstens mit einer Steuereinheit der Blutbehandlungsvorrichtung funktionell derart verbunden, dass insbesondere wenigstens ein von der Recheneinheit erzeugtes Überwachungssignal an die Steuereinheit der Blutbehandlungsvorrichtung zur Regelung und/oder Steuerung wenigstens einer Blutpumpe der Blutbehandlungsvorrichtung und/oder eines Zustands einer arteriellen und/oder venösen Klemme der Blutbehandlungsvorrichtung übermittelbar ist.

In manchen erfindungsgemäßen Ausführungsformen des medizinischen Geräts ist die Blutbehandlungsvorrichtung, insbesondere eine Hämodialysiervorrichtung, eine Hämofiltrationsvorrichtung oder eine Hämodiafiltrationsvorrichtung und/oder beispielsweise als Übernacht-Dialysevorrichtung und/oder Heim-Hämodialysevorrichtung (HHD) ausgebildet und/oder konfiguriert.

In einigen erfindungsgemäßen Ausführungsformen weist die Blutbehandlungsvorrichtung eine Schutzvorrichtung auf, durch die die für Blutbehandlungen geltenden Sicherheitsvorschriften erfüllt werden.

In manchen erfindungsgemäßen Ausführungsformen ist die Schutzvorrichtung und/oder wenigstens eine Regel- und/oder Steuereinrichtung anhand eines ermittelten, übermittelten und/oder erfassten Überwachungssignals des erfindungsgemäßen Systems zur vorzugsweise automatischen Zustandsveränderung von wenigstens einer, mehrerer oder aller Pumpen und/oder Schlauchklemmen, insbesondere der Blutbehandlungsvorrichtung, ausgebildet und/oder konfiguriert. Eine Zustandsveränderung kann beispielsweise ein Stoppen, ein Verlangsamen, ein Beschleunigen, ein Öffnen und/oder ein Schließen oder ähnliches sein.

In einigen erfindungsgemäßen Ausführungsformen der vorliegenden Erfindung wird kein Blutaustritt und/oder keine Blutung optisch, insbesondere anhand der Farbe von Blut, nachgewiesen und/oder keine Farbbildkamera bereitgestellt und/oder verwendet.

In manchen erfindungsgemäßen Ausführungsformen der vorliegenden Erfindung wird keine Blobanalyse, keine Farbanalyse und/oder kein Wachsen von Mustern bei der Verarbeitung des Referenzbildes und/oder der zusätzlichen Bilder verwendet und/oder durchgeführt.

In einigen erfindungsgemäßen Ausführungsformen der vorliegenden Erfindung erfolgt keine Klassifizierung der Pixel des Referenzbildes und/oder der zusätzlichen Bilder als Blut darstellend oder nicht Blut darstellend.

In manchen erfindungsgemäßen Ausführungsformen der vorliegenden Erfindung wird kein Blut oder Blutfleck nachgewiesen und/oder überwacht.

In manchen erfindungsgemäßen Ausführungsformen der vorliegenden Erfindung werden das Referenzbild und/oder die zusätzlichen Bilder nicht im RGB-Format oder im YUV-Format gespeichert und/oder verarbeitet.

In einigen erfindungsgemäßen Ausführungsformen weist die vorliegende Erfindung keine Erkennungsvorrichtung zur Erkennung von Blut und/oder seiner Farbe und/oder keinen solchen Verfahrensschritt auf.

Einige oder alle erfindungsgemäße Ausführungsformen der vorliegenden Erfindung können einen oder mehrere der oben oder im Folgenden genannten Vorteile aufweisen.

Erfindungsgemäß können Nadeldiskonnektionen vorteilhaft frühzeitig erkannt und ein Warnhinweis oder Alarm gegeben werden, bevor eine gefährliche Blutung und/oder eine Nadeldiskonnektion stattgefunden hat. Somit können gefährliche Situationen vermieden werden und die Sicherheit für den Patienten wird erhöht.

In bestimmten erfindungsgemäßen Ausführungsformen kann vorteilhaft bereits bei einer auffälligen Bewegung des Patienten und/oder bei einer, auch nur teilweisen, Bedeckung des Zugangsbereichs, beispielsweise mit einem Fremdkörper wie beispielsweise eine Bettdecke, ein Warnhinweis oder Alarm herausgegeben werden. Dies ist besonders wichtig, denn in einem solchen Fall kann eine Blutung nicht, oder zumindest schwerer, sichtbar sein.

In einigen erfindungsgemäßen Ausführungsformen wird die Sicherheit des Patienten während einer Blutbehandlung, insbesondere während einer Übernacht- oder HHD-Dialyse (Heim-Hämodialyse) vorteilhaft erhöht.

In bestimmten erfindungsgemäßen Ausführungsformen wird vorteilhaft eine berührungslose Überwachung ermöglicht. Insbesondere entfällt das Verbinden von Kabeln oder Leitungen mit dem Patienten und das Verbinden der Kabel mit einer Überwachungseinrichtung.

In einigen erfindungsgemäßen Ausführungsformen können vorteilhaft große Flächen, insbesondere ein breiter Bereich um die Zugangstelle herum, überwacht werden.

In einigen erfindungsgemäßen Ausführungsformen können vorteilhaft mehrere Blutschlauchleitungen, beispielsweise von einem oder von unterschiedlichen Patienten, gleichzeitig überwacht werden.

In manchen erfindungsgemäßen Ausführungsformen entfällt vorteilhaft die Verwendung und/oder die Anbringung von Einwegartikeln zur Feststellung einer Blutung im Bereich der Zugangsstelle, wie beispielsweise pflasterartige und/oder Klebestreifen-Nässedetektoren.

In einigen erfindungsgemäßen Ausführungsformen kann die Überwachung vorteilhaft auch in einer dunklen Umgebung, beispielsweise nachts und/oder ohne zusätzliche Beleuchtung stattfinden.

In bestimmten erfindungsgemäßen Ausführungsformen kann die Verarbeitung, insbesondere die Analyse der Bilddaten, beispielsweise die Form- und/oder Objekt- und/oder Kanten- und/oder Musteranalyse und/oder die Speicherung des Referenzbildes und/oder der aktuellen Bilder und/oder der entsprechenden daraus erzeugten Daten vorteilhaft vereinfacht und/oder beschleunigt werden, insbesondere durch die Verwendung von speziellen Bildformaten, wie beispielsweise Grauwert-Bilder und/oder binäre-Datenformate.

In manchen erfindungsgemäßen Ausführungsformen entfällt vorteilhaft eine Bilddatenformat-Umwandlung ("pre-processing") vor der Verarbeitung der Bilder, wodurch das Verfahren schneller ablaufen kann.

In manchen erfindungsgemäßen Ausführungsformen kann vorteilhaft wenigstens eine Einrichtung in einer größeren Entfernung zum beobachteten Zugangsbereich, beispielsweise in einem anderen Zimmer als das Zimmer in welchem die Überwachung stattfindet, vorgesehen sein, wodurch die Überwachung vorteilhaft von der Ferne möglich ist.

Es können vorteilhaft mehrere Einrichtungen, insbesondere zur Herausgabe eines wahrnehmbaren Signals, an verschiedenen Orten angebracht werden, wodurch beispielsweise mehrere Personen im Falle eines entsprechenden Warnsignals unterrichtet werden bzw. gewarnt werden können.

Die vorliegende Erfindung wird im Folgenden anhand eines bevorzugten Ausführungsbeispiels und anhand der beigefügten Zeichnungen, in welcher identische Bezugszeichen gleiche oder ähnliche Bauteile bezeichnen, exemplarisch erläutert. In den zum Teil stark vereinfachten Figuren gilt:
- **FIG. 1**: zeigt eine beispielhafte Anordnung eines erfindungsgemäßen Systems zum Überwachen eines Zugangsbereichs eines Patienten; und
- **FIG. 2**: zeigt eine schematische Darstellung einer beispielhaften Ausführungsform des erfindungsgemäßen Verfahrens zum Überwachen eines Zugangsbereichs;
- **FIG. 3**: zeigt eine Aufnahme eines Zugangsbereichs eines Patienten, welche mit einer üblichen Kamera aufgenommen worden ist; und
- **FIG. 4**: zeigt eine Aufnahme des Zugangsbereichs der FIG. 3, welche mit einem erfindungsgemäßen System aufgenommen worden ist.

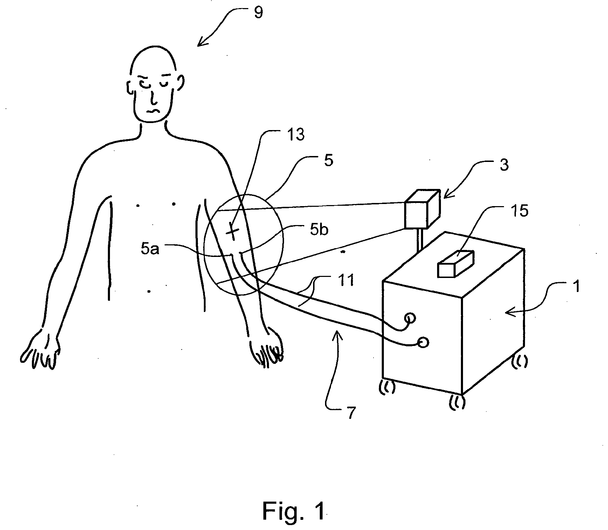

**FIG. 1** zeigt schematisch sehr stark vereinfacht eine erfindungsgemäße Ausführungsform eines Systems und einer Blutbehandlungsvorrichtung 1. Eine Wärmebild- oder Infrarotkamera 3 (auch kurz: Kamera 3), welche hier rein optional an oder auf der Blutbehandlungsvorrichtung 1 montiert ist, ist in der Nähe eines Zugangsbereichs 5 eines extrakorporalen Blutkreislaufs 7 eines Patienten 9 derart bereitgestellt, dass mittels der Kamera 3 der Zugangsbereich 5 beobachtet oder überwacht und Aufnahmen oder Bilder hiervon gemacht werden können.

Im Zugangsbereich 5, welcher als Bereich mit dem Zugang zum Gefäßsystem des Patienten 9 verstanden werden kann, sind im vorliegenden Beispiel zwei Zugangsstellen 5a, 5b und, hier rein optional, zwei Blutschlauchleitungen 11 vorgesehen, welche mit jeweils einer hier nicht dargestellten Patientennadel mit dem Blutkreislauf des Patienten 9 und zugleich mit der Blutbehandlungsvorrichtung 1 verbunden sind.

Der Zugangsbereich 5 weist zudem rein optional eine Orientierungs- oder Skalierungsreferenz, kurz ein Referenzelement 13, auf, das hier rein exemplarisch als kreuzförmiges Element dargestellt ist. Beim Referenzelement 13 kann es sich beispielsweise um eine auf der Haut des Patienten vorhandene, beispielsweise aufgemalte oder auf andere Weise aufgebrachte Markierung handeln. Das Referenzelement kann beispielsweise ein thermisches, beispielsweise kühlendes oder wärmendes Element beliebiger Form sein. In manchen erfindungsgemäßen Ausführungsformen kann das Element elektrisch temperierbar sein, insbesondere kann seine Temperatur dadurch veränderbar und/oder kontrollierbar sein.

Die Kamera 3 kann in einigen Ausführungsformen an anderer Stelle, insbesondere der Blutbehandlungsvorrichtung 1, angeordnet sein. Sie kann ebenfalls optional eine eigene Befestigungsvorrichtung aufweisen. Sie kann beispielsweise einen Antrieb und/oder einen Kipp- und/oder Schwenkmechanismus, aufweisen, vorzugsweise ausgestaltet zum selbsttätigen Folgen eines Referenzelements.

Eine Recheneinheit 15 ist zum Verarbeiten eines Referenzbildes Bref und mit der Wärmebild- oder Infrarotkamera aufgenommenen zusätzlicher Bilder Bzus ausgebildet, programmiert und/oder konfiguriert. Die Recheneinheit 15 kann beispielsweise in oder an der Blutbehandlungsvorrichtung 1 angebracht und/oder mit dieser funktionell verbunden sein. Alternativ kann sich die Recheneinheit 15 an anderer Stelle, beispielsweise in einem anderen Zimmer als der Patient, befinden, und kann insbesondere kabellos mit der Wärmebild- oder Infrarotkamera 3 funktionell verbunden sein.

Auf diese Weise werden von der Recheneinheit 15 Referenzdaten Dref aus dem Referenzbild Bref und zusätzliche Daten Dzus aus den zusätzlichen Bildern Bzus erzeugt. Eine Veränderung der Ausrichtung und/oder der Positionierung wenigstens eines Teils oder Abschnitts der Blutschlauchleitung 11 innerhalb des Zugangsbereichs 5 kann aus den Referenzdaten Dref und den zusätzlichen Daten Dzus nachgewiesen werden, beispielsweise durch Vergleich dieser Daten miteinander. Ein entsprechendes Überwachungssignal ÜS kann erzeugt werden.

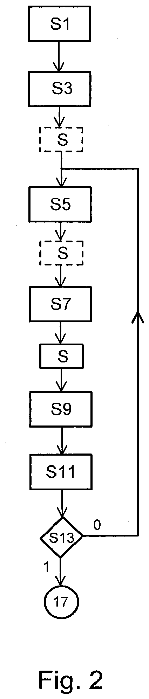

**FIG. 2** zeigt eine beispielhafte Ausführungsform eines erfindungsgemäßen Verfahrens unter Verwendung eines erfindungsgemäßen Systems.

Das Verfahren beinhaltet als Schritt S1, wenigstens eine Wärmebild- oder Infrarotkamera 3 bereitzustellen und derart an einem extrakorporalen Blutkreislauf anzubringen und/oder räumlich anzuordnen, dass die Wärmebild- oder Infrarotkamera 3 die thermische Verteilung des Zugangsbereichs 5 des extrakorporalen Blutkreislaufs 7 beobachten bzw. überwachen kann und Aufnahmen oder Bilder hiervon aufnehmen kann.

Das Verfahren beinhaltet in einem weiteren Schritt S3, die Aufnahme wenigstens eines ersten Bildes des Zugangsbereichs 5 als Referenzbild Bref.

Das Verfahren beinhaltet als weiteren Schritt S5, wenigstens ein zusätzliches Bild Bzus des Zugangsbereichs 5 aufzunehmen.

Das Verfahren beinhaltet als weiteren Schritt S7, das Referenzbild Bref und das wenigstens eine zusätzliche Bild Bzus zu verarbeiten und jeweils daraus Referenzdaten Dref und zusätzliche Daten Dzus zu erzeugen.

Das Verfahren beinhaltet weiterhin als rein optionalen Schritt S, die Referenzdaten Dref und/oder das Referenzbild Bref und/oder die zusätzlichen Daten Dzus und/oder die zusätzlichen Bilder Bzus zu speichern.

In einem weiteren Schritt S9 wird aus den Referenzdaten Dref und den zusätzlichen Daten Dzus eine Änderung der Ausrichtung und/oder der Positionierung wenigstens einer Blutschlauchleitung 11 im Zugangsbereich 5 nachgewiesen oder optional ausgeschlossen. Insbesondere können hierfür die Referenzdaten Dref und die zusätzlichen Daten Dzus miteinander verglichen werden. Beispielsweise kann der Vergleich die Berücksichtigung von, insbesondere vorbestimmbaren, vorbestimmten, voreinstellbaren und/oder voreingestellten, Kriterien, Schwellenwerten, Grenzwerten, logischen Verknüpfungen, Vergleichen und/oder Abfragen beinhalten. Beispielsweise kann als Kriterium definiert werden, dass eine Veränderung, beispielsweise eines zu erkennenden Musters und/oder Objekts, erst berücksichtigt wird und/oder wenigstens eine konkrete Aktion ausgelöst wird, beispielsweise das Anhalten einer Blutpumpe oder das Schließen einer Klemme, wenn die Veränderung außerhalb eines, insbesondere einstellbaren, vorzugsweise vordefinierten, Bereichs oder Rahmens fällt. Damit können insbesondere Fehlalarme vermieden werden und/oder eine Erkennungsgenauigkeit des Verfahrens eingestellt werden. Als Schwellenwerte kommen beispielsweise vordefinierte und/oder einstellbare Längen, Winkel, Flächen, insbesondere welche, die einer bestimmten Temperatur oder einem bestimmten Temperaturbereich zugeordnet sind, oder Verhältnisse hiervon, in Frage.

In einem wiederum weiteren Schritt S11 wird ein entsprechendes Überwachungssignal ÜS erzeugt und/oder verändert, oder nicht.

In einem noch weiteren Schritt S13 wird das Überwachungssignal ÜS abgefragt und entweder an eine weitere Einrichtung 17, beispielsweise an eine Steuereinheit einer Blutbehandlungsvorrichtung und/oder an eine Alarmeinrichtung, übermittelt, sofern beispielsweise eine Veränderung der Ausrichtung und/oder der Positionierung der Blutschlauchleitung nachgewiesen wurde und/oder wenn wenigstens ein zu erkennendes Objekt, beispielsweise eine Blutschlauchleitung oder ein Referenzelement, oder Abschnitte hiervon, nicht erkannt wurde (,1').Andernfalls (,0') werden die Verfahrenschritte S5 bis S13 wiederholt.

Die Schritte S5 bis S11 können, insbesondere während einer Blutbehandlung, beispielsweise mehrfach in der Minute und/oder pro Sekunde, vorzugsweise zweimal pro Sekunde, wiederholt werden.

Damit wird sichergestellt, dass ein Blutverlust innerhalb von weniger als 60s, beispielsweise von weniger als 30s oder 10s, erkannt, wird und dass entsprechende, insbesondere automatische, Gegenmaßnahmen, wie beispielsweise das Anhalten bestimmter Pumpen und/oder das Schließen bestimmter Schlauchklemmen, insbesondere vor Erreichung eines kritischen Blutverlusts, eingeleitet werden können. Bei einem typischen Blutfluss von 400 ml/min kann innerhalb dieser Zeit ein kritischer Blutverlust, der in der Regel ca. 400 ml betragen dürfte, vorteilhaft vermieden werden.

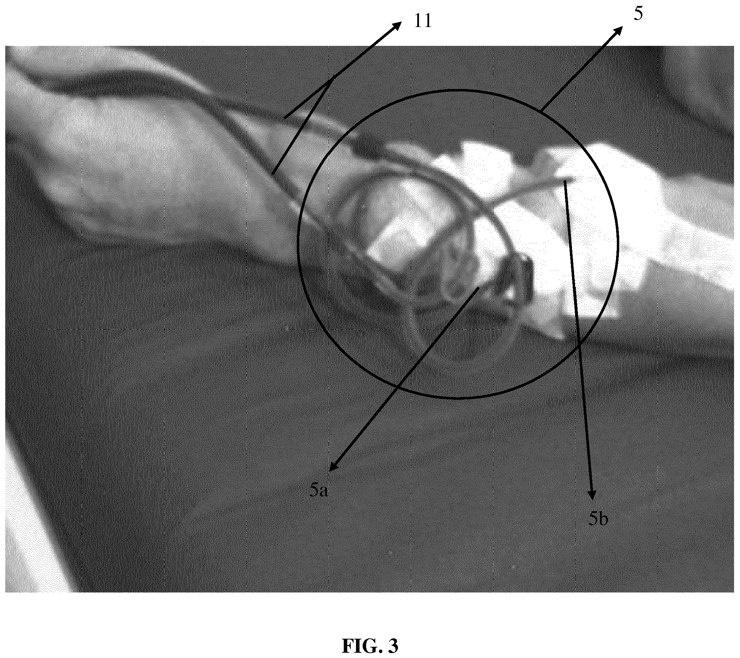

**FIG. 3** zeigt eine Aufnahme eines Zugangsbereichs eines Patienten, die mit üblichen Aufnahmemitteln, beispielsweise mit einer normalen Kamera aufgenommen wurde.

Zu erkennen sind unter anderem ein Zugangsbereich 5 eines Patienten mit zwei Zugangstellen 5a, 5b zweier Blutschlauchleitungen 11, welche mit einer nicht dargestellten Blutbehandlungsvorrichtung 1 verbunden und/oder verbindbar sind.

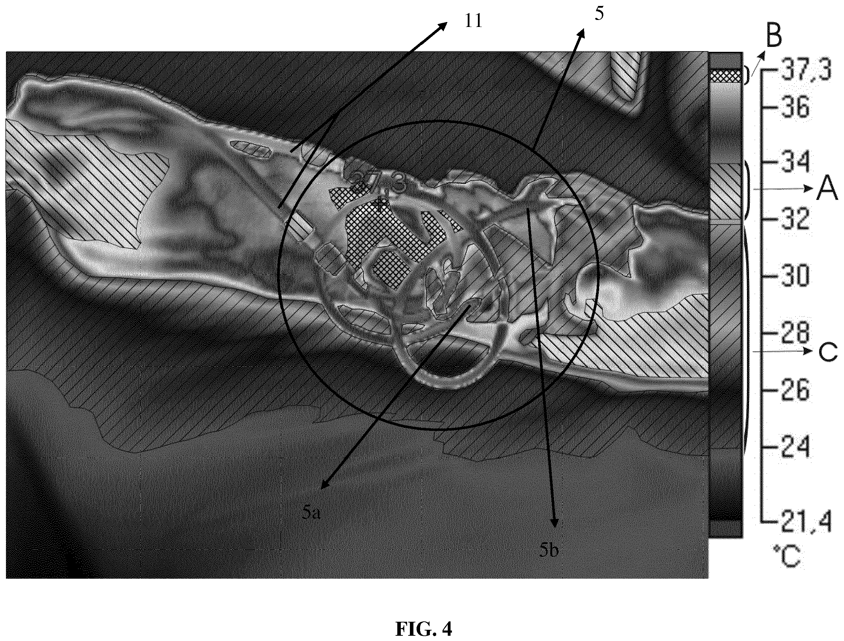

**FIG. 4** zeigt eine mit einer Wärmebild- oder Infrarotkamera und/oder mit einem erfindungsgemäßen System aufgenommene Aufnahme des Zugangsbereichs 5 der FIG. 3.

Dabei zeigen die mit A bezeichneten und durch eine um -45° geneigte Schraffierung definierten Flächen Oberflächenbereiche mit einer Oberflächentemperatur von ca. um die 33°C. Die mit B bezeichneten und durch eine Kreuzschraffierung definierten Flächen zeigen dagegen Oberflächenbereiche mit einer Oberflächentemperatur von ca. um die 37°C. Die mit C bezeichneten und durch eine um 45° geneigte Schraffierung definierten Flächen zeigen dagegen Oberflächenbereiche mit einer Oberflächentemperatur von ca. 24°C bis 32°C.

Deutlich zu erkennen ist, dass die Blutschlauchleitungen 11 des extrakorporalen Blutkreislaufs im Zugangsbereich 5, insbesondere ihre Ausrichtung und/oder Positionierung, beispielsweise gegenüber Flächen, die eine andere Oberflächentemperatur darstellen (beispielsweise die mit A, B, C bezeichneten Flächen oder andere hier nicht dargestellten Flächen), besonders gut, auch vom Hintergrund, zu erkennen sind.

Ein Austritt von Blut, beispielsweise verursacht bei einer Ruptur im Schlauchsystem, an defekten Konnektoren und/oder an einer gelösten Katheterverbindung, kann einen Blutfleck, das heißt eine auftretende warme Fläche, verursachen und somit eine erkennbare Veränderung der Oberflächentemperaturverteilung, wobei auch spritzendes Blut als warmes Element außerhalb der definierten Flächen zu erkennen sein kann.

Zudem geht in der Regel einer Nadel- und/oder Schlauchleitungen-Diskonnektion eine, gegebenenfalls auffällige, Änderung der Ausrichtung und/oder Positionierung zumindest eines Teils wenigstens einer der Blutschlauchleitungen 11 im Zugangsbereich 5 voraus.

Durch wiederholte Aufnahmen des Zugangsbereichs 5 und anschließende Auswertung der Temperaturverteilungsinformation der Oberfläche mit dem erfindungsgemäßen System und/oder mit dem erfindungsgemäßen Verfahren kann daher eine effiziente Überwachung des Zugangsbereichs 5, insbesondere der Ausrichtung und/oder Positionierung der Blutschlauchleitungen 11, im Zugangsbereich 5, erreicht werden.

Somit können Diskonnektionen vorteilhaft leichter, unabhängig von den tatsächlichen Lichtverhältnissen und/oder frühzeitig erkannt werden, so dass sie mit geeigneten Maßnahmen begegnet werden können, wodurch gefährliche Situationen vermieden werden und die Sicherheit für den Patienten erhöht wird.

**Bezugszeichenliste**

| Bezugszeichen | Beschreibung |
|---|---|
| 1 | Blutbehandlungsvorrichtung, |
| 3 | Wärmebild- oder Infrarotkamera, Kamera |
| 5 | Zugangsbereich |
| 5a, 5b | Zugangsstellen |
| 7 | extrakorporaler Blutkreislauf |
| 9 | Patient |
| 11 | Blutschlauchleitung |
| 13 | Referenzelement |
| 15 | Recheneinheit |
| 17 | Einrichtung |

## Patentansprüche

1. Verfahren zur Überwachung der Ausrichtung und/oder Positionierung wenigstens einer mit einer arteriellen und/oder venösen Patientennadel verbundenen Blutschlauchleitung (11), oder wenigstens eines Abschnitts der Blutschlauchleitung (11), eines extrakorporalen Blutkreislaufs (7), in einem Zugangsbereich (5) oder einem Überwachungsbereich, wobei das Verfahren wenigstens umfasst:
- Bereitstellen wenigstens einer Wärmebild- oder Infrarotkamera (3) zur Beobachtung der Temperaturverteilung der Oberfläche des Zugangsbereichs (5);
- Aufnehmen wenigstens eines ersten Bildes des Zugangsbereichs (5) als Referenzbild;
- Aufnehmen wenigstens eines zusätzlichen Bildes des Zugangsbereichs (5);
- Verarbeiten des wenigstens einen Referenzbildes und des wenigstens einen zusätzlichen Bildes mittels einer Recheneinheit (15), welche ausgebildet, programmiert und/oder konfiguriert ist, um erste Daten aus dem Referenzbild als Referenzdaten und zusätzliche Daten aus dem zusätzlichen Bild zu erzeugen,
wobei zumindest die Referenzdaten und/oder das Referenzbild gespeichert werden, **dadurch gekennzeichnet, dass** die Recheneinheit (15) zusätzlich programmiert, ausgebildet und/oder konfiguriert ist, um aus den Referenzdaten und den zusätzlichen Daten eine Veränderung der Ausrichtung und/oder der Positionierung wenigstens eines Abschnitts der Blutschlauchleitung (11) im Zugangsbereich (5) nachzuweisen und ein entsprechendes Überwachungssignal zu erzeugen oder zu verändern.

2. Verfahren nach Anspruch 1, wobei die Verarbeitung des Referenzbildes und/oder der zusätzlichen Bilder wenigstens Muster- und/oder Objekterkennung, Formanalyse, Formextraktion, Regions-Erkennung und/oder Kantenerkennung umfasst.

3. Verfahren nach einem der vorhergehenden Ansprüche, wobei die Recheneinheit (15) wenigstens zur Erkennung eines vorgegebenen und/oder vorgebbaren Objektes in einem Referenzbild und/oder in einem zusätzlichen Bild ausgebildet, programmiert und/oder konfiguriert ist.

4. Verfahren nach Anspruch 2 oder 3, wobei die Recheneinheit (15) zur Ermittlung der Anzahl der Pixel wenigstens einer Region und/oder eines Objektes des Referenzbildes und/oder des zusätzlichen Bildes konfiguriert ist, wobei die Pixel einem vorbestimmten oder vorbestimmbaren Temperaturbereich zugeordnet sind.

5. Verfahren nach einem der Ansprüche 2 bis 4, wobei die Recheneinheit (15) zur Erkennung eines zuvor angebrachten oder vorhandenen Referenzelements (13) ausgebildet, programmiert und/oder konfiguriert ist.

6. Verfahren nach einem der Ansprüche 2 bis 5, wobei die Recheneinheit (15) ferner ausgebildet, programmiert und/oder konfiguriert ist, um wenigstens ein Überwachungssignal zu erzeugen oder zu verändern, wenn ein Referenzelement (13) nicht erkannt wird und/oder wenn nicht wenigstens ein Abschnitt eines zu erkennenden Objektes erkannt wird.

7. Verfahren nach einem der vorhergehenden Ansprüche, wobei das Überwachungssignal wenigstens an eine Einrichtung übermittelt wird, welche wenigstens zur Ausgabe eines wahrnehmbaren Alarms ausgebildet ist.

8. Verfahren nach einem der vorhergehenden Ansprüche, wobei das Überwachungssignal darstellbare Bilddaten wenigstens des Bildes umfasst, bei welchem das Überwachungssignal erzeugt und/oder geändert wurde.

9. Verfahren nach einem der vorhergehenden Ansprüche, wobei der Nachweis oder die Feststellung der Veränderung der Ausrichtung und/oder Positionierung wenigstens eines Abschnitts der wenigstens einen Blutschlauchleitung (11) im Zugangsbereich (5) durch einen Vergleich der Referenzdaten mit den zusätzlichen Daten erfolgt.

10. Verfahren nach einem der vorhergehenden Ansprüche, wobei die Referenzdaten und/oder die zusätzlichen Daten wenigstens ein Muster, eine Anzahl von erkannten Objekten, Abmessungen und/oder Flächen von erkannten Objekten, und/oder eine relative Position und/oder Ausrichtung und/oder wenigstens einen Abstand zwischen wenigstens zwei erkannten Objekten und/oder zwischen wenigstens einem Objekt und einem Referenzelement (13) umfassen.

11. Verfahren nach einem der vorhergehenden Ansprüche, wobei die Referenzdaten und/oder die zusätzlichen Daten wenigstens die Ausrichtung und/oder Positionierung wenigstens einer Blutschlauchleitung (11), oder wenigstens eines Abschnitts hiervon, bezüglich einer weiteren Blutschlauchleitung (11), oder wenigstens eines Abschnitts hiervon, umfasst.

12. System mit wenigstens einer Wärmebild- oder Infrarotkamera (3) **gekennzeichnet durch** wenigstens eine Recheneinheit (15) konfiguriert und/oder programmiert zur Ausführung eines Verfahrens nach einem der vorhergehenden Ansprüche 1 bis 11.

13. Blutbehandlungsvorrichtung (1) **gekennzeichnet durch** wenigstens ein System nach Anspruch 12.

14. Blutbehandlungsvorrichtung (1) nach Anspruch 13, welche wenigstens einen mit einem Blutkreislauf eines Patienten (9) verbindbaren extrakorporalen Blutkreislauf (7) aufweist oder welche mit einem solchen verbunden und/oder verbindbar ist.

15. Blutbehandlungsvorrichtung (1) nach vorhergehendem Anspruch 14, wobei das System mit wenigstens einer Steuereinheit der Blutbehandlungsvorrichtung (1) funktionell derart verbunden ist, dass ein von der Recheneinheit (15) erzeugtes Überwachungssignal an die Steuereinheit der Blutbehandlungsvorrichtung (1) zur Regelung und/oder Steuerung wenigstens einer Blutpumpe der Blutbehandlungsvorrichtung (1) und/oder eines Zustands einer arteriellen und/oder venösen Klemme der Blutbehandlungsvorrichtung (1) übermittelbar ist.

## Claims

1. A method of monitoring the orientation and/or the positioning of at least one blood tube line (11) connected to an arterial and/or to a venous patient needle, or at least of a portion of the blood tube line (11), of an extracorporeal blood circuit (7), in an access area (5) or in a monitoring area, wherein the method comprises at least:
- providing at least one thermal imaging or infrared camera (3) for observing the temperature distribution of the surface of the access area (5);
- recording at least one first image of the access area (5) as a reference image;
- recording at least one additional image of the access area (5);
- processing the at least one reference image and the at least one additional image by means of a computing unit (15), which is designed, programmed and/or configured to generate first data from the reference image as reference data and additional data from the additional image,
wherein at least the reference data and/or the reference image are stored, **characterized in that** the computing unit (15) is additionally programmed, designed and/or configured to detect, from the reference data and from the additional data, a change in the orientation and/or the positioning of at least one portion of the blood tube line (11) in the access area (5) and to generate or modify a corresponding monitoring signal.

2. The method according to claim 1, wherein the processing of the reference image and/or of the additional images comprises at least pattern and/or object recognition, shape analysis, shape extraction, region and/or edge recognition.

3. The method according to anyone of the preceding claims, wherein the computing unit (15) is designed, programmed and/or configured at least for recognizing a predetermined and/or predefinable object in a reference image and/or in an additional image.

4. The method according to claim 2 or 3, wherein the computing unit (15) is configured to determine the number of pixels of at least one region and/or one object of the reference image and/or of the additional image, wherein the pixels are associated with a predetermined or predeterminable temperature range.

5. The method according to anyone of claims 2 to 4, wherein the computing unit (15) is designed, programmed and/or configured to recognize a previously attached or existing reference element (13).

6. The method according to anyone of claims 2 to 5, wherein the computing unit (15) is further designed, programmed and/or configured to generate or modify at least one monitoring signal when a reference element (13) is not recognized and/or when at least one portion of an object to be recognized is not recognized.

7. The method according to anyone of the preceding claims, wherein the monitoring signal is transmitted at least to a device which is at least designed to output a perceptible alarm.

8. The method according to anyone of the preceding claims, wherein the monitoring signal comprises displayable image data of at least the image from which the monitoring signal has been generated and/or modified.

9. The method according to anyone of the preceding claims, wherein the detection or the determination of the change in the orientation and/or in the positioning of at least one portion of the at least one blood tube line (11) in the access area (5) takes place by means of a comparison of the reference data with the additional data.

10. The method according to anyone of the preceding claims, wherein the reference data and/or the additional data comprise at least one pattern, a number of recognized objects, dimensions and/or areas of recognized objects, and/or a relative position and/or orientation and/or at least one distance between at least two recognized objects and/or between at least one object and a reference element (13).

11. The method according to anyone of the preceding claims, wherein the reference data and/or the additional data comprise at least the orientation and/or the positioning of at least one blood tube line (11) or at least a portion thereof, with respect to another blood tube line (11), or at least a portion thereof.

12. A system comprising at least one thermal imaging camera or infrared camera (3) **characterized by** at least one computing unit (15) configured and/or programmed for carrying out a method according to anyone of the preceding claims 1 to 11.

13. A blood treatment apparatus (1) **characterized by** at least one system according to claim 12.

14. The blood treatment apparatus (1) according to claim 13, comprising at least an extracorporeal blood circuit (7) connectable to a blood circuit of a patient (9) or which is connected and/or connectable with such one.

15. The blood treatment apparatus (1) according to the preceding claim 14, wherein the system is operatively connected to at least one control device of the blood treatment apparatus (1), such that a monitoring signal generated by the computing unit (15) can be transmitted to the control device of the blood treatment apparatus (1) for regulating and/or controlling at least one blood pump of the blood treatment apparatus (1) and/or a state of an arterial and/or venous clamp of the blood treatment apparatus (1).

## Revendications

1. Un procédé de surveillance de l'orientation et/ou du positionnement d'au moins un conduit sanguin en tuyau souple (11) connecté à une aiguille artérielle et/ou veineuse d'un patient, ou d'au moins une partie du conduit sanguin en tuyau souple (11), d'un circuit sanguin extracorporel (7), dans une zone d'accès (5) ou dans une zone de surveillance, le procédé comprenant au moins:
- la fourniture d'au moins une caméra d'imagerie thermique ou infrarouge (3) pour l'observation de la répartition thermique de la surface de la zone d'accès (5);
- l'enregistrement d'au moins une première image de la zone d'accès (5) en tant qu'image de référence;
- l'enregistrement d'au moins une image supplémentaire de la zone d'accès (5);
- le traitement d'au moins l'image de référence et d'au moins une image supplémentaire au moyen d'une unité de calcul (15) conçue, programmée et/ou configurée pour générer des premières données à partir de l'image de référence en tant que données de référence et des données supplémentaires à partir de l'image supplémentaire,
où au moins les données de référence et/ou l'image de référence sont stockées, **caractérisé en ce que** l'unité de calcul (15) est en outre programmée, conçue et/ou configurée de façon à détecter, à partir des données de référence et des données supplémentaires, un changement d'orientation et/ou de positionnement d'au moins une partie du conduit sanguin en tuyau souple (11) dans la zone d'accès (5) et à générer ou modifier un signal de surveillance correspondant.

2. Le procédé selon la première revendication, où le traitement de l'image de référence et/ou des images supplémentaires comprend au moins une reconnaissance de motif et/ou d'objet, une analyse de forme, une extraction de forme, une reconnaissance de région et/ou de contour.

3. Le procédé selon l'une quelconque des revendications précédentes, où l'unité de calcul (15) est conçue, programmée et/ou configurée de façon à au moins reconnaître un objet prédéterminé et/ou prédéfinissable dans une image de référence et/ou dans une image supplémentaire.

4. Le procédé selon la revendication 2 ou 3, où l'unité de calcul (15) est configurée de façon à déterminer le nombre de pixels d'au moins une région et/ou d'un objet de l'image de référence et/ou de l'image supplémentaire, les pixels étant associés à une plage de températures prédéterminée ou prédéterminable.

5. Le procédé selon l'une quelconque des revendications 2 à 4, où l'unité de calcul (15) est conçue, programmée, et/ou configurée de façon à reconnaître un élément de référence (13) préalablement attaché ou existant.

6. Le procédé selon l'une quelconque des revendications 2 à 5, où l'unité de calcul (15) est en outre conçue, programmée et/ou configurée de façon à générer ou à modifier au moins un signal de surveillance lorsqu'un élément de référence (13) n'est pas reconnu et/ou lorsqu'au moins une partie d'un objet à reconnaître n'est pas reconnue.

7. Le procédé selon l'une quelconque des revendications précédentes, où le signal de surveillance est transmis au moins à un dispositif conçu au moins pour émettre une alarme perceptible.

8. Le procédé selon l'une quelconque des revendications précédentes, où le signal de surveillance comprend des données d'image affichables d'au moins de l'image pour laquelle le signal de surveillance a été généré et/ou modifié.

9. Le procédé selon l'une quelconque des revendications précédentes, où la détection ou la détermination du changement d'orientation et/ou de positionnement d'au moins une partie d'au moins un conduit sanguin de tuyau souple (11) dans la zone d'accès (5) se fait par comparaison des données de référence avec les données supplémentaires.

10. Le procédé selon l'une quelconque des revendications précédentes, où les données de référence et/ou les données supplémentaires comprennent au moins un motif, un nombre d'objets reconnus, des dimensions et/ou des surfaces d'objets reconnus et/ou une position et/ou une orientation relative (s) et/ou au moins une distance entre au moins deux objets reconnus et/ou entre au moins un objet et un élément de référence (13) .

11. Le procédé selon l'une quelconque des revendications précédentes, où les données de référence et/ou les données supplémentaires comprennent au moins l'orientation et le positionnement d'au moins un conduit sanguin de tuyau souple (11) ou d'au moins une partie de celui-ci, par rapport à un autre conduit sanguin de tuyau souple (11), ou au moins à une partie de cellui-ci.

12. Un système comprenant au moins une caméra d'imagerie thermique ou une caméra infrarouge (3) **caractérisé par** au moins une unité de calcul (15) configurée et/ou programmée afin de mettre en oeuvre un procédé selon l'une quelconque des précédentes revendications 1 à 11.

13. Un appareil de traitement du sang (1) **caractérisé par** au moins un système selon la revendication 12.

14. L'appareil de traitement du sang (1) selon la revendication 13, comprenant au moins un circuit sanguin extracorporel (7) connectable à un circuit sanguin d'un patient (9) ou étant connecté et/ou connectable à un tel circuit.

15. L'appareil de traitement du sang (1) selon la revendication 14, où le système est connecté de manière fonctionnelle à au moins une unité de contrôle de l'appareil de traitement du sang (1), de sorte qu'un signal de surveillance généré par l'unité de calcul (15) puisse être transmis à l'unité de contrôle de l'appareil de traitement du sang (1) afin de réguler et/ou de commander au moins une pompe à sang de l'appareil de traitement du sang (1) et/ou un état d'une pince artérielle et/ou veineuse de l'appareil de traitement du sang (1).
